# EUROPEAN PATENT SPECIFICATION

(11) **EP 2 432 548 B1**
(45) Date of publication and mention of the grant of the patent: **31.08.2016**
(21) Application number: 10777212.1
(22) Date of filing: 17.05.2010
(51) Int. Cl.: A61N 1/04, H01R 4/48

(54) **ELECTRODE ASSEMBLY**
ELEKTRODENANORDNUNG
ENSEMBLE ÉLECTRODE

(30) Priority: 18.05.2009 AU 2009902232
(43) Date of publication of application: 28.03.2012
(73) Proprietor: Impedimed Limited, Pinkenba, QLD 4008 (AU)
(72) Inventor: GAW, Richelle Leanne, Greenslopes Queensland 4120 (AU); KIRSCHEN, Todd, Fullerton California 92831 (US)
(74) Representative: Appleyard Lees IP LLP
(86) International application number: PCT/AU2010/000575
(87) International publication number: WO 2010/132923

(56) References cited:
- GB-A- 1 441 622
- US-A- 3 868 165
- US-A- 4 922 911
- US-A- 6 101 413
- US-A1- 2002 163 408
- US-A1- 2005 117 196
- US-B2- 6 623 312
- US-B2- 7 270 580

## Description

### Background of the Invention

The present invention relates to an electrode assembly for use with a subject, an electrical connector for coupling an electrical cable to an electrode assembly, and an electrode system including an electrode assembly and an electrical connector.

### Description of the Prior Art

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

It is known to provide electrode assemblies for coupling a subject to an electrical system, such as a ECG (electrocardiogram) or impedance measuring device.

Traditionally electrodes are formed from conductive pads of an electrolytic gel provided on a substrate. An electrical connection with the conductive pad is then achieved using a connector such as a crocodile clip. However, this arrangement suffers a number of disadvantages. For example, the electrical connection between the clip and pad can be unreliable, resulting in inaccurate measurements. Additionally, when electrodes are positioned individually on a subject, this can result in variations in electrode position between different measurements, which can in turn impact on accuracy of resulting measurements.

WO2007089278 describes an electrode assembly for use with a living subject. The electrode assembly includes a substrate having first and second openings extending therethrough, and a first terminal at least partially received within the first opening. The first terminal includes an end portion having a first size. At least a portion of the first terminal configured to conduct electrical current. A second terminal is at least partially received within the second opening. The second terminal includes an end portion having a second size that is different to the first size of the first terminal end portion. At least a portion of the second terminal is configured to conduct electrical current. The assembly also includes a first electrolytic element configured to transfer electrical current between the skin of a living subject the first terminal, and a second electrolytic element configured to transfer electrical current between the skin of a living subject and the second terminal.

US 4,922,911 discloses a similar electrode assembly comprising at least one movable substrate portion formed by cut outs.

Whilst the use of the substrate with apertures ensures a fixed separation between electrodes, the use of separate terminals that have to be mounted within apertures of the substrate results in the need to manufacture multiple components, and then assemble these into a final assembly. This in turn increases manufacturing requirements and hence costs.

### Summary of the Present Invention

The present invention seeks to substantially overcome, or at least ameliorate, one or more disadvantages of existing arrangements.

The inventive electrode assembly is defined in claim 1.

Typically the substrate includes two substrate portions, at least part of each terminal being provided on a respective substrate portion.

Each substrate portion is arranged so that the terminals face in a common direction when extending away from the substrate.

Typically an end of the at least one substrate portion is hinged with respect to the substrate. Typically a join between each side of the at least one substrate portion and the substrate is perforated to allow the joins to be torn, thereby allowing the at least one substrate portion to be moved.

Typically the at least one substrate portion includes an aperture associated with each terminal, the aperture being adapted to cooperate with a projection on the connector, to thereby facilitate positioning of the terminal relative to a conductor of an electrical connector.

Typically the conductive elements and the terminals are mounted on a first side of the substrate, the substrate portion being adapted to allow the terminals to extend outwardly from a second opposing side of the substrate.

Typically at least part of a first side of the substrate is adhesive to thereby adhere the substrate to the subject in use.

Typically the conductive elements are spaced apart in a first direction, and the conductive elements extend along the substrate in a second direction perpendicular to the first direction. Typically the conductive elements are spaced apart a distance selected dependent on the intended use of the electrode assembly.

Typically the first conductive element is longer than the second conductive element.

Typically the substrate is flexible, thereby allowing the substrate to conform to the subject in use.

Typically the terminal includes a layer of conductive ink applied to the substrate.

Typically each conductive element includes:
a) a layer of conductive ink applied to the substrate; and,
b) a conductive gel applied to the conductive ink.

In a second broad form the present invention provides an electrical connector for coupling an electrical cable to an electrode assembly, the connector including:
a) a housing for receiving the electrical cable;
b) at least two conductors mounted to the housing and coupled to the electrical cable in use; and,
c) a biasing member for biasing a terminal against each of the conductors to thereby electrically couple the conductors to the terminals.

Typically the connector includes at least two projections mounted to the housing, each projection being associated with a respective conductor, each projection being adapted to cooperate with an aperture associated with each terminal to thereby facilitate positioning of the terminal relative to the conductor.

Typically the biasing member includes:
a) an arm mounted to the housing, the arm being moveable between open and closed positions; and,
b) a retaining element for retaining the arm in the closed position to thereby urge each terminal against the respective conductor.

Typically the arm includes two resilient members, each resilient member being for urging a terminal against a respective conductor.

Typically the housing defines a cavity, the connector including processing electronics mounted in the cavity, the processing electronics being coupled to the conductors and being coupled to the cable in use.

Typically the processing electronics includes:
a) a sensor; and,
b) a signal generator.

It will be appreciated that the broad forms of the invention may be used individually or in combination, and may be used for performing a range of electrical measurements of different subjects.

### Brief Description of the Drawings

An example of the present invention will now be described with reference to the accompanying drawings, in which: -
Figure 1A is a schematic plan view of an example of an electrode assembly;
Figure 1B is a schematic side view of the electrode assembly of Figure 1A;
Figure 2A is a schematic perspective front view of an example of an electrical connector;
Figure 2B is a schematic perspective front view of the electrical connector of Figure 2A with a biasing member in an open position;
Figure 2C is a schematic perspective rear view of the electrical connector of Figure 2A;
Figure 2D is a schematic perspective exploded view of the electrical connector of Figure 2A;
Figure 3A is a schematic front view of an example of an electrode system including the electrode assembly of Figure 1A and the electrical connector of Figure 2A;
Figure 3B is a schematic end view of the electrode system of Figure 3A;
Figure 4 is a schematic diagram of an example of an impedance measuring device;
Figure 5 is a schematic diagram of an example of an electrode system processing electronics incorporating a signal generator and a sensor.

### Detailed Description of the Preferred Embodiments

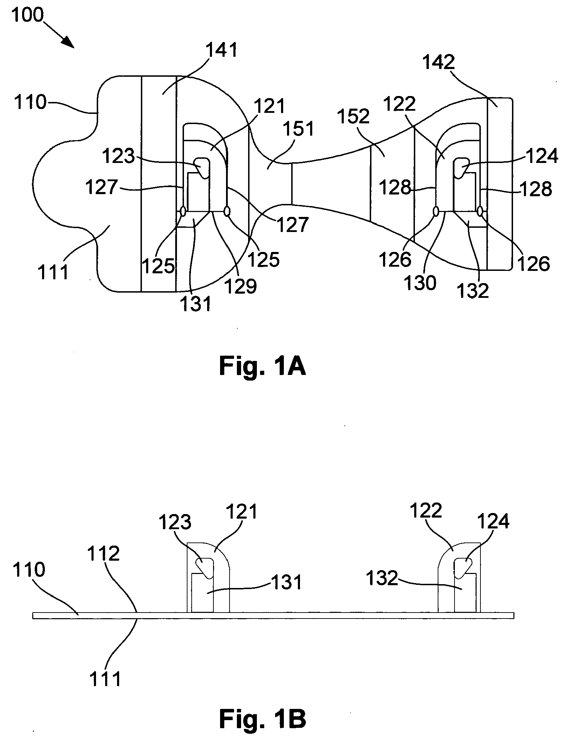

An example of an electrode assembly for use with the subject will now be described with reference to Figures 1A and 1B.

In this example, the electrode assembly 100 includes a substrate 110 having a first side 111 and a second side 112, and two substrate portions 121, 122. Each substrate portion 121, 122 includes at least part of a terminal 131, 132, which is in turn electrically connected to a respective conductive element 141, 142. In use, the conductive elements 141, 142 act as electrodes, allowing electrical contact to be made with a subject. The substrate portions 121, 122, are moveable with respect to the substrate 110, to allow the terminals to extend outwardly from the second side 112, as shown in Figure 1B. This enables the terminals to be connected via an appropriate electrical connector, to a measuring device, thereby allowing measurements to be made.

Whilst two substrate portions are shown in this example, this is not essential, and alternatively, both terminals may be mounted on a common substrate portion.

In one example, the substrate portions 121, 122 are formed by providing perforations 127, 128 along opposing sides of each substrate portion 121, 122, with one end of the substrate portions being unconnected, so that the substrate portions can be hinged along an end 129, 130 where the substrate portion adjoins the substrate, after the perforations 127, 128 have been torn. Cut-outs 125, 126 are typically provided where the perforated sides 127, 128 adjoin the ends 129, 130 so that as the perforations are torn, the tear stops when the cut-outs 125, 126 are reached, thereby preventing the substrate 110 being torn in use.

In use, when the electrode assembly is to be used, the substrate portions 121, 122 are torn free of the substrate and hinged about the ends 129, 130 allowing the terminals 131, 132 to be raised clear of the second side of the substrate 112 as shown in Figure 1B.

The substrate portions 121, 122 also typically include apertures 123, 124 which in use cooperate with projections to align the terminals with an electrical connector as will be described in more detail below.

The substrate 110 may also include adhesive strips 151, 152 allowing the substrate to be mounted on the subject, although this is not essential and any suitable mounting method, such as straps or the like, may be used. The substrate 110 may also include markings to assist with positioning the electrode assembly 100 on a subject.

The electrodes 141, 142 are typically spaced in a first direction by a fixed distance which may be selected dependent on an intended use of the electrode assembly 100. Thus, for example, if the electrode assembly 100 is to be used for Bioimpedance measurements, a different separation may be used compared to if the electrode assembly is to be used for ECG measurements.

The electrodes typically extend in a second direction perpendicular to the first direction. The length of the electrodes may also depend on the intended use. Thus, in the case of performing Bioimpedance measurements, it is typical for the electrode 141 to act as a drive electrode and therefore be longer and hence greater in area that the electrode 142, which acts as a sense electrode. The reason for this is that the accuracy of an impedance measurement is typically susceptible to the contact impedance of the drive electrode, which is minimised by the use of a larger area electrode. In contrast the area of a sense electrode is less important, and can therefore be made smaller to assist in comfortable application to the subject.

The substrate 110, terminals 131, 132 and electrodes 141, 142 are typically formed from flexible materials that are able to conform to the shape of part of the user, in use. This ensures good electrical connectivity between the conductive elements and the subject, thereby helping to ensure accuracy of resulting measurements, whilst also ensuring the electrode assembly is comfortable in use.

The substrate 110 is typically formed from a biologically inactive material to prevent unwanted interactions with the subject. The substrate material is also typically electrically insulative to prevent leakage between the electrodes. Accordingly the substrate 110 may be manufactured from any suitable material such as a plastic or the like.

The terminals 131, 132 are typically formed from layers of conductive ink applied to the substrate 110, with the electrodes 141, 142 typically formed from a layer of conductive ink, overlayed with a conductive gel. It will be appreciated from this that during manufacture, the electrode assembly can be formed by printing the conductive ink onto the substrate 110 to form both the terminals and the underlay of the electrode, with the electrically conductive gel then being applied to form the electrodes 141, 142. Following, or prior to this process, a punching procedure can be used to punch out the apertures 123, 124 as well as the cut-outs 125, 126 and to define the required perforations 127, 128. This process can also occur simultaneously with the preparation of the shape of the substrate. Thus, in a single step, the substrate may be punched out of a larger piece of substrate material with the substrate portions and apertures being created at the same time.

It will be appreciated from the above, that the electrode assembly can be manufactured rapidly and cheaply, thereby allowing the electrode assembly to be provided as a disposable product.

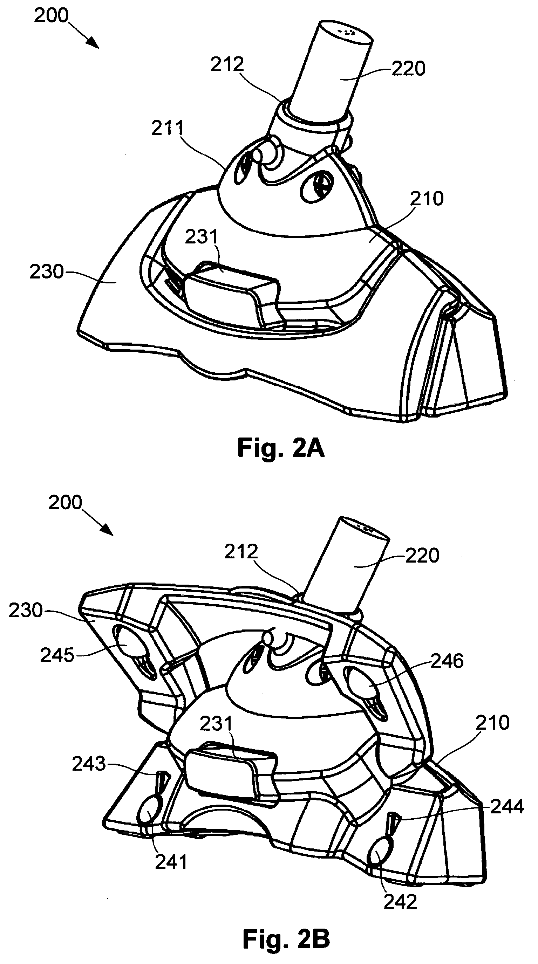

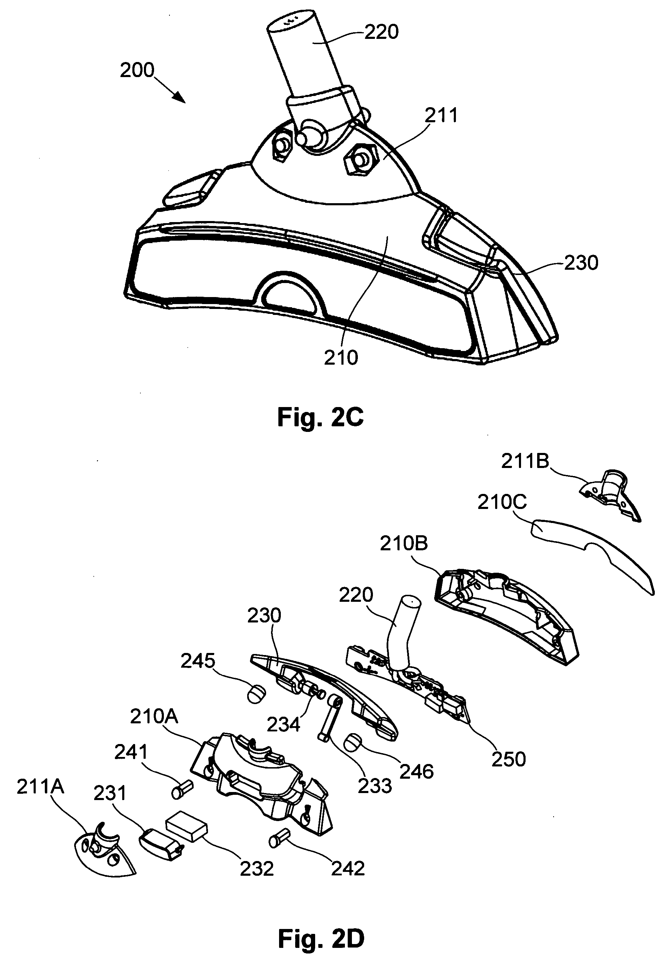

In use, the electrode assembly is typically coupled to an electrical cable, via an electrical connector, to provide onward connectivity to a measuring device. An example electrical connector will now be described with reference to Figures 2A to 2B.

In this example, the electrical connector 200 includes a housing 210 having at least two conductors 241, 242 mounted thereto, which in use are electrically connected to an electrical cable 220. The electrical connector also includes a biasing member 230, which in this example is in the form of an arm, pivotally mounted to the housing 210. In use, the biasing member 230 urges the terminals 131, 132 of the electrode assembly against the conductors 241, 242, thereby providing electrical contact between the electrodes 141, 142 and the cable 220.

In this example, the arm 230 can be moved between a closed position shown in Figure 2A and an open position shown in Figure 2B. Movement of the arm 230 is typically constrained by a retaining member 231 which operates to retain the arm 230 in the closed position until the retaining member 231 is released.

Further features of the electrical connectors will now be described.

In this example, the housing includes a clamp 211 defining an aperture 212, which operates to retain the cable in position.

Positioned on the housing adjacent to the conductors 241, 242 are respective associated projections 243, 244, which facilitate in aligning the terminals 131, 132 with the conductors 241, 242. The biasing member 230 typically also includes resilient members 245, 246 which in one example are in the form of rubber spheres. The resilient members align with the conductors 241, 242 to assist with ensuring adequate connection between the conductors 241, 242 and the terminals 131, 132 of the electrode assembly 100, as will be described in more detail below.

As shown in the exploded view of Figure 2D, the housing 210 is typically formed from two housing portions 210A, 210B which define a cavity. In use, the cavity can contain processing electronics, shown generally at 250, which are connected to the cable 220. The conductors 241, 242 extend through apertures in the housing portion 210A and then contact a respective part of the processing electronics, allowing connection between the conductors 241, 242 and the cable.

In this example, the housing also includes a fascia plate 210C which is used to cover any physical connectors, such as bolts which may be used to couple the housing portions 210A, 210B together. In one example, the fascia plate is in the form of a label that may also be used for providing printed instructions or symbols to indicated correct use or orientation of the connector.

The biasing member 230 is pivotally mounted coupled to the housing 210 via a pivot, such as an axle 234, with a spring 233 being used to bias the retaining member 230 into the open position. A retaining element 231 is provided in the form of a push button, which is mounted on a deformable stop 232. The retaining element 231 cooperates with the biasing member to retain the biasing member 230 in the closed position. In use, the retaining element 231 can be pushed inwards, allowing the biasing member 230 to be released so that it moves into the open position.

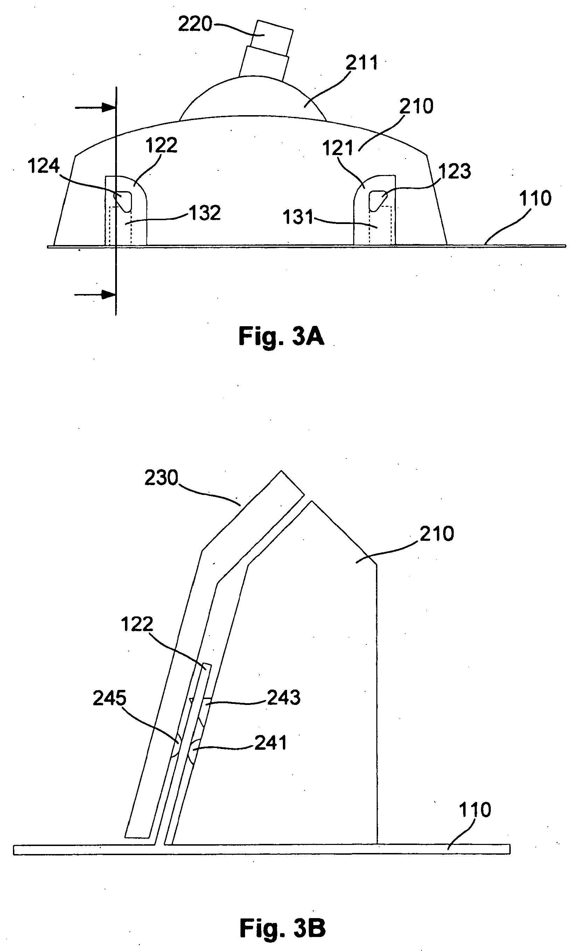

An example of the connection of the electrode assembly to the electrical connector, to thereby form an electrode system is shown in Figures 3A and 3B. In this example, it is assumed that the first side of the electrode assembly is mounted on the subject using the adhesive strips 151, 152, with the substrate portions 121, 122 extending away from the second side of the substrate 110 as shown in Figure 1B.

In this example, the retaining member 231 is depressed, thereby releasing the biasing member 230, which moves to the open position under the action of the spring 233. The substrate portions 121, 122 are then positioned relative to the housing 210 so that the projections 243, 244 extend through the apertures 123, 124. At this point the conductors 241, 242 are aligned with the terminals 131, 132. The biasing member 230 may then be returned to the closed position so that the resilient members 245, 246 are urged against the second side of the substrate portions 121, 122 thereby urging the terminals 131, 132 against the conductors 241, 242.

It will be appreciated by a person skilled in the art that this has a number of benefits. Firstly, the use of the rubber resilient members ensures good electrical contact between the terminals 131, 132 and the corresponding conductors 241, 242. Secondly, because the terminals 131, 132 are both mounted on the same side of the substrate portions 121, 122, both terminals 131, 132 face in a common direction. This ensures that a respective one of the terminals 131, 132 aligns with a respective one of the conductors 241, 242. This can in turn be used to ensure that a particular one of the electrodes 141, 142 is electrically connected to a corresponding one of the conductors 241, 242. In the event that respective ones of the electrodes 141, 142 are to be used for a particular purpose, such as if one of the electrodes is to be used as a drive electrode and the other a sense electrode, then this can ensure that electrical signals are applied to the correct electrodes.

The above described electrode arrangement can be used in any scenario in which electrical contact with a subject is required. In one example, the electrodes are used in performing bioimpedance measurements.

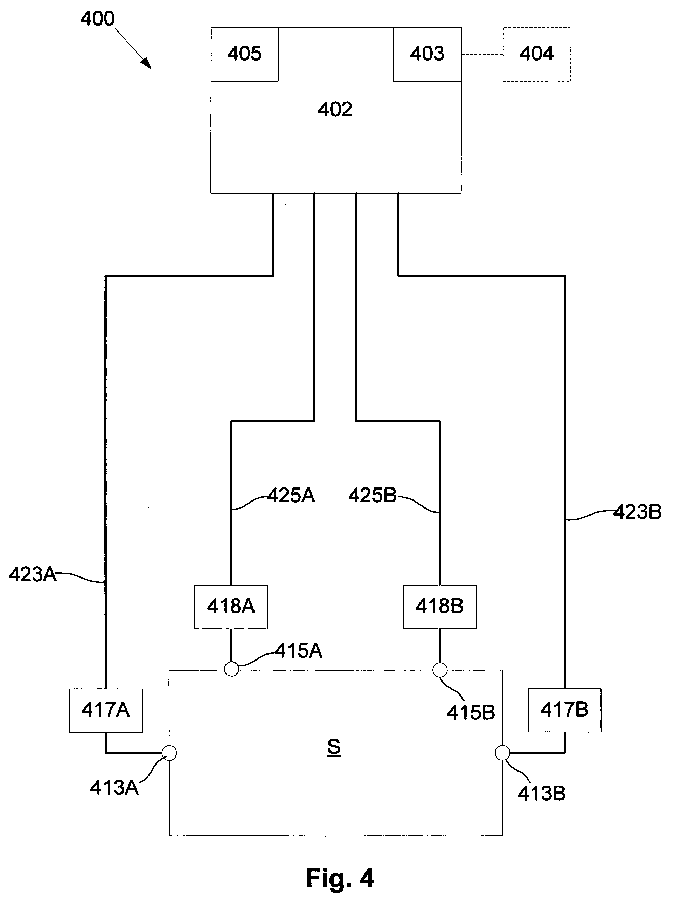

An example of apparatus suitable for performing an analysis of a subject's bioelectric impedance will now be described with reference to Figure 4.

As shown the apparatus includes a measuring device 400 including a processing system 402, connected to one or more signal generators 417A, 417B, via respective first leads 423A, 423B, and to one or more sensors 418A, 418B, via respective second leads 425A, 425B. The connection may be via a switching device, such as a multiplexer, although this is not essential.

In use, the signal generators 417A, 417B are coupled to two first electrodes 413A, 413B, which therefore act as drive electrodes to allow signals to be applied to the subject S, whilst the one or more sensors 418A, 418B are coupled to the second electrodes 415A, 415B, which act as sense electrodes, allowing signals across the subject S to be sensed. Typically, each pair of first and second electrodes 417A, 418A, 417B, 418B, would be formed from electrodes 141, 142 provided on an electrode assembly 100 as described above.

The signal generators 417A, 417B and the sensors 418A, 418B may be provided at any position between the processing system 402 and the electrodes 413A, 413B, 415A, 415B, and may be integrated into the measuring device 400. However, in one example, the signal generators 417A, 417B and the sensors 418A, 418B form part of the processing electronics 250 integrated into the electrical connector 200, with the leads 423A, 423B, 425A, 425B connecting the signal generators 417A, 417B and the sensors 418A, 418B to the processing system 402 forming part of the cables 220.

It will be appreciated that the above described system is a two channel device utilising two of the electrical connectors 200 and two electrode assemblies 100, to perform a classical four-terminal impedance measurement, with each channel being designated by the suffixes A, B respectively. The use of a two channel device is for the purpose of example only, and additional channels may be used.

An optional external interface 403 can be used to couple the measuring device 400, via wired, wireless or network connections, to one or more peripheral devices 404, such as an external database or computer system, barcode scanner, or the like. The processing system 402 will also typically include an I/O device 405, which may be of any suitable form such as a touch screen, a keypad and display, or the like.

In use, the processing system 402 is adapted to generate control signals, which cause the signal generators 417A, 417B to generate one or more alternating signals, such as voltage or current signals of an appropriate waveform, which can be applied to a subject S, via the first electrodes 413A, 413B. The sensors 418A, 418B then determine the voltage across or current through the subject S, using the second electrodes 415A, 415B and transfer appropriate signals to the processing system 402.

Accordingly, it will be appreciated that the processing system 402 may be any form of processing system which is suitable for generating appropriate control signals and at least partially interpreting the measured signals to thereby determine the subject's bioelectrical impedance, and optionally determine other information such as the presence, absence or degree of conditions, such as oedema, lymphoedema, measures of body composition, cardiac function, or the like.

The processing system 402 may therefore be a suitably programmed computer system, such as a laptop, desktop, PDA, smart phone or the like. Alternatively the processing system 102 may be formed from specialised hardware, such as an FPGA (field programmable gate array), or a combination of a programmed computer system and specialised hardware, or the like.

In use, the electrode assemblies 100 are positioned on the subject, and coupled to corresponding electrical connectors 200, to allow one or more signals to be injected into the subject S. The location of the first electrodes will depend on the segment of the subject S under study. Thus, for example, the first electrodes 413A, 413B can be placed on the thoracic and neck region of the subject S to allow the impedance of the chest cavity to be determined for use in cardiac function analysis. Alternatively, positioning electrodes on the wrist and ankles of a subject allows the impedance and hence fluid levels in the limbs and/or the entire body to be determined, for use in oedema analysis, or the like.

Once the electrodes are positioned, one or more alternating signals are applied to the subject S, via the first leads 423A, 423B and the first electrodes 413A, 413B. The nature of the alternating signal will vary depending on the nature of the measuring device and the subsequent analysis being performed.

For example, the system can use Bioimpedance Analysis (BIA) in which a single low frequency signal is injected into the subject S, with the measured impedance being used directly in the determination of biological parameters, such as extracellular fluid levels, which can be indicative of total body water, oedema or the like. In one example, the signal has a frequency of below 40 kHz.

In contrast Bioimpedance Spectroscopy (BIS) devices perform impedance measurements at multiple frequencies over a selected frequency range. Whilst any range of frequencies may be used, typically frequencies range from very low frequencies (4 kHz) to higher frequencies (15000 kHz). Similarly, whilst any number of measurements may be made, in one example the system can use 256 or more different frequencies within this range, to allow multiple impedance measurements to be made within this range.

When impedance measurements are made at multiple frequencies, these can be used to derive one or more impedance parameter values, such as values of *R₀, Z_{c}, R_{∞},* which correspond to the impedance at zero, characteristic and infinite frequencies. These can in turn be used to determine information regarding both intracellular and extracellular fluid levels.

Thus, the measuring device 400 may either apply an alternating signal at a single frequency, at a plurality of frequencies simultaneously, or a number of alternating signals at different frequencies sequentially, depending on the preferred implementation. The frequency or frequency range of the applied signals may also depend on the analysis being performed.

In one example, the applied signal is generated by a voltage generator, which applies an alternating voltage to the subject S, although alternatively current signals may be applied. In one example, the voltage source is typically symmetrically arranged, with each of the signal generators 417A, 417B being independently controllable, to allow the signal voltage across the subject to be varied.

A voltage difference and/or current is measured between the second electrodes 415A, 415B. In one example, the voltage is measured differentially, meaning that each sensor 418A, 418B is used to measure the voltage at each second electrode 415A, 415B and therefore need only measure half of the voltage as compared to a single ended system.

The acquired signal and the measured signal will be a superposition of voltages generated by the human body, such as the ECG (electrocardiogram), voltages generated by the applied signal, and other signals caused by environmental electromagnetic interference. Accordingly, filtering or other suitable analysis may be employed to remove unwanted components.

The acquired signals are then used to determine first and second parameter values, such as resistance and reactance values, at each frequency. In one example, this is achieved using an algorithm to derive an amplitude and phase signal at each frequency, with these values in turn being used to derive the resistance and reactance values.

As part of the above described process, the distance between the second electrodes 415A, 415B may be measured and recorded. Similarly, other parameters relating to the subject may be recorded, such as the height, weight, age, sex, health status, any interventions and the date and time on which they occurred. Other information, such as current medication, may also be recorded. This can then be used in performing further analysis of the impedance measurements, so as to allow determination of the presence, absence or degree of oedema, to assess body composition, or the like.

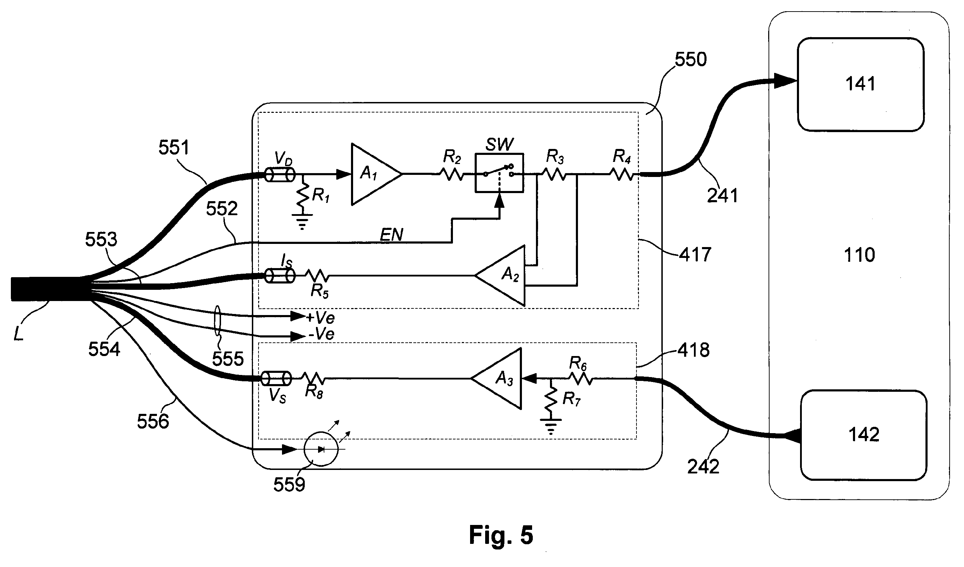

An example of the processing electronics 250 will now be described with reference to Figure 5.

In this example, the processing electronics 250 incorporates a substrate 550, such as a printed circuit board (PCB), or the like, having the respective signal generator 417 and sensor 418 mounted thereon. The general functionality of the signal generator 417 and sensor 418 are represented by the components shown. In practice a greater number of components may be used in a suitable arrangement, as would be appreciated by persons skilled in the art, and the components shown are merely intended to indicate the functionality of the signal generator and the sensor 417,418.

The signal generator 417 and the sensor 418 are coupled via the conductors 241, 242 to the electrodes 141, 142.

In this example, the signal generator 417 includes an amplifier *A₁* having an input coupled to a cable 551. The input is also coupled to a reference voltage, such as ground, via a resistor *R₁*. An output of the amplifier *A₁* is connected via a resistor *R₂*, to a switch *SW,* which is typically a CMOS (complementary metal-oxide semiconductor) switch or a relay that is used to enable the voltage source. The switch *SW* is controlled via enabling signals *EN* received from the processing system 102 via a cable 552.

The switch *SW* is in turn coupled via two resistors *R₃, R₄,* arranged in series, and then, via the cable conductor 241, to the electrode 141. A second amplifier *A₂* is provided with inputs in parallel with the first of the two series resistor *R₃* and with an output coupled via a resistor *R₅,* to a cable 553.

It will be appreciated from the above that the cables 551, 552, 553 therefore forms the lead 423 of Figure 4. A range of different resistor values may be used, but in one example, the resistors have values of *R₁* = *R₂* = *R₅* = 50Ω, and *R₃* = *R₄* = 100Ω.

The sensor 418 generally includes an amplifier *A₃* having an input connected via a resistor *R₆,* to the conductor 242. The input is also coupled via a resistor *R₇,* to a reference voltage such as a ground. An output of the amplifier *A₃* is coupled to a cable 554, via a resistor *R₈*.

It will be appreciated from the above that the cable 554 therefore forms the lead 425 of Figure 4. A range of different resistor values may be used, but in one example, the resistors have values of *R₆* = 100Ω, *R₇* = 10MΩ and, *R₈* = 50Ω.

Optional power cables 555 can be provided for supplying power signals +*Ve,* -*Ve,* for powering the signal generator 417 and the sensor 418, although alternatively an on board power source such as a battery, may be used. Additionally, a cable 556 may be provided to allow an LED 557 to be provided on the substrate 550. This can be controlled by the processing system 402, allowing the operating status of the electrode system to be indicated.

Operation of the signal generator 417 and the sensor 418 will now be described in more detail. For the purpose of this explanation, the voltage drive signal, current signal and sensed voltage will be generally indicated as *V_{D}, I_{S}, V_{S},* and in practice, these would be equivalent to respective ones of the voltage drive signals, current signals and sensed voltages *V_{DA}, V_{DB}, I_{SA}, I_{SB}, V_{SA}, V_{SB}* in the example above.

In use, the amplifier *A₁* operates to amplify an analogue voltage signal received from the processing system 402 and apply this to the subject S via the conductor 241, so that the applied voltage drive signal *V_{D}* drives a current signal *I_{S}* through the subject S. The voltage drive signal *V_{D},* will only be applied if the switch *SW* is in a closed position and the switch *SW* can therefore be placed in an open position to isolate the voltage source from the subject S. This may be used if a pair of drive and sense electrodes 141, 142 are being used to sense voltages only, and are not being used to apply a voltage drive signal *V_{D}* to the subject S. Isolating the signal generator 417 from the drive electrode 413 removes the unintended return current path(s) that would otherwise be present due to the low output impedance of the amplifier *A₁,* thereby constraining current to flow only between the two selected drive electrodes 413. Other techniques may be used to achieve a similar effect, such as using an amplifier incorporating a high impedance output-disable state.

The current signal *I_{S}* being applied to the subject S is detected and amplified using the amplifier *A₂,* with the amplified current signal *I_{S}* being returned to the processing system 402, along the cable 553.

Similarly, the sensor 418 operates by having the amplifier *A₃* amplify the voltage detected at the second electrode 415, returning the amplified analogue sensed voltage signal *V_{S}* along the cable 554, to the processing system 402.

Accordingly, it will be appreciated that above described measuring device can be used together with an electrode system including the electrode assembly of Figure 1A and the electrical connector of Figure 2A, to thereby allow bioimpedance measurements to be performed.

Persons skilled in the art will appreciate that numerous variations and modifications will become apparent. All such variations and modifications which become apparent to persons skilled in the art, should be considered to fall within the spirit and scope that the invention broadly appearing before described.

The term impedance is intended to cover any form of impedance measurement including resistance, reactance or admittance measurements.

## Claims

1. An electrode assembly (100) for use with a subject, the assembly (100) including:
a substrate (110) including at least one moveable substrate portion (121, 122); and,
first and second terminals (131, 132), each terminal (131, 132) being electrically coupled to a respective conductive element (141, 142), and at least part of each terminal (131, 132) being provided on the at least one moveable substrate portion (121, 122), such that movement of the substrate portion (121, 122) allows at least part of the terminal (131, 132) to extend away from the substrate (110) to thereby facilitate connection of the terminal to a respective connector, each substrate portion (121, 122) being arranged so that the terminals (131, 132) are mounted on a same side of the substrate portions (121, 122),
**characterised in that** each substrate portion (121, 122) is arranged so that the terminals (131, 132) face in the same direction when extending away from the substrate (110), wherein the respective conductive element (141,142) are first and second conductive elements (141,142) provided on the substrate (110), the first and second conductive elements (141,142) being spaced apart, and being adapted to provide an electrical connection to the subject.

2. An electrode assembly (100) according to claim 1, wherein the substrate includes two substrate portions (121, 122), at least part of each terminal (131, 132) being provided on a respective substrate portion (121, 122).

3. An electrode assembly (100) according to any one of the claims 1 to 2, wherein an end of the at least one substrate portion (121, 122) is hinged with respect to the substrate (110).

4. An electrode assembly (100) according to any one of the claims 1 to 3, wherein a join (127, 128) between each side of the at least one substrate portion (121, 122) and the substrate (110) is perforated to allow the joins (127, 128) to be torn, thereby allowing the at least one substrate portion (121, 122) to be moved.

5. An electrode assembly (100) according to any one of the claims 1 to 4, wherein the at least one substrate portion (121, 122) includes an aperture (123, 124) associated with each terminal (131, 132), the aperture (123, 124) being adapted to cooperate with a projection on the connector, to thereby facilitate positioning of the terminal (131, 132) relative to a conductor of an electrical connector.

6. An electrode assembly (100) according to any one of the claims 1 to 5, wherein the conductive elements (141, 142) and the terminals (131, 132) are mounted on a first side (111) of the substrate (110), the substrate portion (121, 122) being adapted to allow the terminals (131, 132) to extend outwardly from a second opposing side (112) of the substrate (110).

7. An electrode assembly (100) according to any one of the claims 1 to 6, wherein at least part of a first side (111) of the substrate (110) is adhesive to thereby adhere the substrate (110) to the subject in use.

8. An electrode assembly (100) according to any one of the claims 1 to 7, wherein the conductive elements (141, 142) are spaced apart in a first direction, and wherein the conductive elements (141, 142) extend along the substrate (110) in a second direction perpendicular to the first direction.

9. An electrode assembly (100) according to claim 1 to 8, wherein the conductive elements (141, 142) are spaced apart a distance selected dependent on the intended use of the electrode assembly (100).

10. An electrode assembly (100) according to any one of the claims 1 to 9, wherein the first conductive element (141) is longer than the second conductive element (142).

11. An electrode assembly (100) according to any one of the claims 1 to 10, wherein the substrate (110) is flexible, thereby allowing the substrate (110) to conform to the subject in use.

12. An electrode assembly (100) according to any one of the claims 1 to 11, wherein the terminal (131, 132) includes a layer of conductive ink applied to the substrate (110).

13. An electrode assembly (100) according to any one of the claims 1 to 12, wherein each conductive element (141, 142) includes:
a) a layer of conductive ink applied to the substrate (110); and,
b) a conductive gel applied to the conductive ink.

14. An electrode assembly (100) according to any one of the claims 1 to 13, wherein an end of each substrate portion (121, 122) is hinged with respect to the substrate (110), the hinges (129, 130) being collinear.

15. An electrode system for electrically coupling a subject to an electrical cable (220), the system including:
a) an electrode assembly (100) according to any one of the claims 1 to 14; and,
b) a connector (200) including:
i) a housing (210) for receiving the electrical cable (220);
ii) at least two conductors (241, 242) mounted to the housing (210) and coupled to the electrical cable (220) in use; and,
iii) a biasing member (230) for biasing a terminal (131, 132) against each of the conductors (241, 242) to thereby electrically couple the conductors (241, 242) to the terminals (131, 132).

## Patentansprüche

1. Elektrodenanordnung (100) zur Verwendung mit einem Patienten, wobei die Anordnung (100) Folgendes umfasst:
ein Substrat (110), das mindestens einen beweglichen Substratabschnitt (121, 122) aufweist; und
einen ersten und zweiten Anschluss (131, 132), wobei jeder Anschluss (131, 132) mit einem jeweiligen leitfähigen Element (141, 142) elektrisch gekoppelt ist und mindestens ein Teil jedes Anschlusses (131, 132) an dem mindestens einen beweglichen Substratabschnitt (121, 122) vorgesehen ist, so dass es eine Bewegung des Substratabschnitts (121, 122) ermöglicht, dass sich mindestens ein Teil des Anschlusses (131, 132) vom Substrat (110) weg erstreckt, um dadurch eine Verbindung des Anschlusses mit einem jeweiligen Verbinder zu unterstützen, wobei jeder Substratabschnitt (121, 122) so angeordnet ist, dass die Anschlüsse (131, 132) auf einer selben Seite der Substratabschnitte (121, 122) angebracht sind,
**dadurch gekennzeichnet, dass** jeder Substratabschnitt (121, 122) so angeordnet ist,
dass die Anschlüsse (131, 132) in dieselben Richtung weisen, wenn sie sich vom Substrat (110) weg erstrecken,
wobei das jeweilige leitfähige Element (141,142) aus einem ersten und zweiten leitfähigen Element (141,142) besteht, die auf dem Substrat (110) vorgesehen sind, wobei das erste und zweite leitfähige Element (141,142) in einem Abstand angeordnet und eingerichtet sind, eine elektrische Verbindung mit dem Patienten bereitzustellen.

2. Elektrodenanordnung (100) nach Anspruch 1, wobei das Substrat zwei Substratabschnitte (121, 122) aufweist, wobei mindestens ein Teil jedes Anschlusses (131, 132) an einem jeweiligen Substratabschnitt (121, 122) vorgesehen ist.

3. Elektrodenanordnung (100) nach einem der Ansprüche 1 bis 2, wobei ein Ende des mindestens einen Substratabschnitts (121, 122) bezüglich des Substrats (110) um ein Scharnier drehbar ist.

4. Elektrodenanordnung (100) nach einem der Ansprüche 1 bis 3, wobei eine Verbindung (127, 128) zwischen jeder Seite des mindestens einen Substratabschnitts (121, 122) und des Substrats (110) perforiert ist, um es zu ermöglichen, dass die Verbindungen (127, 128) zerrissen werden, wodurch es ermöglicht wird, dass der mindestens eine Substratabschnitt (121, 122) bewegt wird.

5. Elektrodenanordnung (100) nach einem der Ansprüche 1 bis 4, wobei der mindestens eine Substratabschnitt (121, 122) eine Öffnung (123, 124) aufweist, die mit jedem Anschluss (131, 132) verknüpft ist, wobei die Öffnung (123, 124) eingerichtet ist, mit einem Vorsprung am Verbinder zusammenzuwirken, um dadurch die Positionierung des Anschlusses (131, 132) relativ zu einem Leiter eines elektrischen Verbinders zu unterstützen.

6. Elektrodenanordnung (100) nach einem der Ansprüche 1 bis 5, wobei die leitfähigen Elemente (141, 142) und der Anschluss (131, 132) auf einer ersten Seite (111) des Substrats (110) angebracht sind, wobei der Substratabschnitt (121, 122) eingerichtet ist, es den Anschlüssen (131, 132) zu ermöglichen, sich von einer zweiten gegenüberliegenden Seite (112) des Substrats (110) zu erstrecken.

7. Elektrodenanordnung (100) nach einem der Ansprüche 1 bis 6, wobei mindestens ein Teil einer ersten Seite (111) des Substrats (110) klebend ist, um dadurch das Substrat (110) im Gebrauch an den Patienten zu kleben.

8. Elektrodenanordnung (100) nach einem der Ansprüche 1 bis 7, wobei die leitfähigen Elemente (141, 142) in eine erste Richtung in einem Abstand angeordnet sind, und wobei sich die leitfähigen Elemente (141, 142) längs des Substrats (110) in eine zur ersten Richtung senkrechte zweite Richtung erstrecken.

9. Elektrodenanordnung (100) nach Anspruch 1 bis 8, wobei die leitfähigen Elemente (141, 142) in einem Abstand angeordnet sind, der abhängig von der beabsichtigten Verwendung der Elektrodenanordnung (100) ausgewählt ist.

10. Elektrodenanordnung (100) nach einem der Ansprüche 1 bis 9, wobei das erste leitfähige Element (141) länger ist als das zweite leitfähige Element (142).

11. Elektrodenanordnung (100) nach einem der Ansprüche 1 bis 10, wobei das Substrat (110) flexibel ist, wodurch es dem Substrat (110) ermöglicht wird, sich im Gebrauch an den Patienten anzupassen.

12. Elektrodenanordnung (100) nach einem der Ansprüche 1 bis 11, wobei der Anschluss (131, 132) eine Schicht einer leitfähigen Tinte aufweist, die das Substrat (110) aufgetragen ist.

13. Elektrodenanordnung (100) nach einem der Ansprüche 1 bis 12, wobei jedes leitfähige Element (141, 142) aufweist:
a) eine Schicht einer leitfähigen Tinte, die das Substrat (110) aufgetragen ist; und
b) ein leitfähiges Gel, das auf die leitfähige Tinte aufgetragen ist.

14. Elektrodenanordnung (100) nach einem der Ansprüche 1 bis 13, wobei ein Ende jedes Substratabschnitts (121, 122) bezüglich des Substrats (110) um ein Scharnier drehbar ist, wobei die Scharniere (129, 130) kollinear sind.

15. Elektrodensystem zum elektrischen Koppeln eines Patienten an eine elektrische Leitung (220), wobei das System aufweist:
a) eine Elektrodenanordnung (100) nach einem der Ansprüche 1 bis 14; und,
b) einen Verbinder (200), der aufweist:
i) ein Gehäuse (210) zum Aufnehmen der elektrischen Leitung (220);
ii) mindestens zwei Leiter (241, 242), die am Gehäuse (210) angebracht und im Gebrauch mit der elektrischen Leitung (220) gekoppelt sind; und,
iii) ein Vorspannelement (230) zum Vorspannen eines Anschlusses (131, 132) gegen jeden der Leiter (241, 242), um dadurch die Leiter (241, 242) mit den Anschlüssen (131, 132) elektrisch zu koppeln.

## Revendications

1. Ensemble électrode (100) destiné à une utilisation avec un sujet, l'ensemble (100) incluant :
un substrat (110) incluant au moins une portion de substrat mobile (121, 122) ;
des première et deuxième bornes (131, 132), chaque borne (131, 132) étant couplée électriquement à un élément conducteur respectif (141, 142), et au moins une partie de chaque borne (131, 132) étant prévue sur ladite au moins une portion de substrat mobile (121, 122), de sorte qu'un mouvement de la portion de substrat (121, 122) permette à au moins une partie de la borne (131, 132) de s'étendre pour s'éloigner du substrat (110) afin de faciliter ainsi la connexion de la borne à un connecteur respectif, chaque portion de substrat (121, 122) étant agencée de sorte que les bornes (131, 132) sont montées sur un même côté des portions de substrat (121, 122),
**caractérisé en ce que** chaque portion de substrat (121, 122) est agencée de sorte que les bornes (131, 132) sont dirigées dans le même sens lorsqu'elles s'étendent pour s'éloigner du substrat (110),
l'élément conducteur respectif (141, 142) étant des premier et deuxième éléments conducteurs (141, 142) prévus sur le substrat (110), les premier et deuxième éléments conducteurs (141, 142) étant espacés l'un de l'autre, et étant conçus pour procurer une connexion électrique vers le sujet.

2. Ensemble électrode (100) selon la revendication 1, le substrat incluant deux portions de substrat (121, 122), au moins une partie de chaque borne (131, 132) étant prévue sur une portion de substrat respective (121, 122).

3. Ensemble électrode (100) selon l'une quelconque des revendications 1 à 2, une extrémité de ladite au moins une portion de substrat (121, 122) étant en articulation par rapport au substrat (110).

4. Ensemble électrode (100) selon l'une quelconque des revendications 1 à 3, un lien (127, 128) entre chaque côté de ladite au moins une portion de substrat (121, 122) et le substrat (110) étant perforé afin de permettre aux liens (127, 128) d'être déchirés, ce qui permet ainsi à ladite au moins une portion de substrat (121, 122) d'être déplacée.

5. Ensemble électrode (100) selon l'une quelconque des revendications 1 à 4, ladite au moins une portion de substrat (121, 122) incluant une ouverture (123, 124) associée à chaque borne (131, 132), l'ouverture (123, 124) étant conçue pour agir en coopération avec une saillie sur le connecteur, afin de faciliter ainsi le positionnement de la borne (131, 132) relativement à un conducteur d'un connecteur électrique.

6. Ensemble électrode (100) selon l'une quelconque des revendications 1 à 5, les éléments conducteurs (141, 142) et les bornes (131, 132) étant montés sur un premier côté (111) du substrat (110), la portion de substrat (121, 122) étant conçue pour permettre aux bornes (131, 132) de s'étendre vers l'extérieur à partir d'un deuxième côté opposé (112) du substrat (110).

7. Ensemble électrode (100) selon l'une quelconque des revendications 1 à 6, au moins une partie d'un premier côté (111) du substrat (110) étant adhésive afin de faire adhérer ainsi le substrat (110) au sujet lors de l'utilisation.

8. Ensemble électrode (100) selon l'une quelconque des revendications 1 à 7, les éléments conducteurs (141, 142) étant espacés l'un de l'autre dans un premier sens, et les éléments conducteurs (141, 142) s'étendant le long du substrat (110) dans un deuxième sens lequel est perpendiculaire au premier sens.

9. Ensemble électrode (100) selon la revendication 1 à 8, les éléments conducteurs (141, 142) étant espacés l'un de l'autre d'une distance sélectionnée en fonction de l'utilisation envisagée de l'ensemble électrode (100).

10. Ensemble électrode (100) selon l'une quelconque des revendications 1 à 9, le premier élément conducteur (141) étant plus long que le deuxième élément conducteur (142).

11. Ensemble électrode (100) selon l'une quelconque des revendications 1 à 10, le substrat (110) étant flexible, ce qui permet ainsi au substrat (110) de suivre la forme du sujet lors de l'utilisation.

12. Ensemble électrode (100) selon l'une quelconque des revendications 1 à 11, la borne (131, 132) incluant une couche d'encre conductrice laquelle est appliquée au substrat (110).

13. Ensemble électrode (100) selon l'une quelconque des revendications 1 à 12, chaque élément conducteur (141, 142) incluant :
a) une couche d'encre conductrice laquelle est appliquée au substrat (110) ; et
b) un gel conducteur lequel est appliqué à l'encre conductrice.

14. Ensemble électrode (100) selon l'une quelconque des revendications 1 à 13, une extrémité de chaque portion de substrat (121, 122) étant en articulation par rapport au substrat (110), les charnières (129, 130) étant colinéaires.

15. Système d'électrodes pour coupler électriquement un sujet à un câble électrique (220), le système incluant :
a) un ensemble électrode (100) selon l'une quelconque des revendications 1 à 14 ; et,
b) un connecteur (200) incluant :
i) un logement (210) pour recevoir le câble électrique (220) ; ;
ii) au moins deux conducteurs (241, 242) montés sur le logement (210) et couplés au câble électrique (220) lors de l'utilisation ; et,
iii) un élément de sollicitation (230) pour solliciter une borne (131, 132) contre chacun des conducteurs (241, 242) afin de coupler ainsi électriquement les conducteurs (241, 242) aux bornes (131, 132).
